Europäisches Patentamt

European Patent Office

Office européen des brevets

(19)

(11) Veröffentlichungsnummer: **0 140 231**
**A2**

(12) # EUROPÄISCHE PATENTANMELDUNG

(21) Anmeldenummer: **84112108.0**

(22) Anmeldetag: **10.10.84**

(51) Int. Cl.⁴: **C 07 C 125/03**

(30) Priorität: **19.10.83 DE 3337939**

(43) Veröffentlichungstag der Anmeldung:
**08.05.85 Patentblatt 85/19**

(84) Benannte Vertragsstaaten:
**CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Hagemann, Hermann, Dr.**
**Kandinskystrasse 52**
**D-5090 Leverkusen 1(DE)**

(72) Erfinder: **Lenthe, Manfred, Dr.**
**Michaelshöhe 40**
**D-5068 Odenthal(DE)**

(72) Erfinder: **Döring, Fritz**
**Am Hang 13**
**D-5068 Odenthal(DE)**

(72) Erfinder: **Mohrmann, Karl-Heinrich, Dr.**
**Roonstrasse 61**
**D-5600 Wuppertal 1(DE)**

(54) **Verfahren zur Herstellung von N-Chlorcarbonyl-isocyaniddichlorid.**

(57) N-Chlorcarbonyl-isocyaniddichlorid wird durch Umsetzung von Methylisocyanat mit Chlor unter Zusatz von Lösungsmitteln und in Gegenwart eines organischen, radikalbildenden Stoffes hergestellt.

EP 0 140 231 A2

BAYER AKTIENGESELLSCHAFT  5090 Leverkusen, Bayerwerk

Konzernverwaltung RP
Patentabteilung  ⌐ Gai/Kü-c  18. 10. 83

Verfahren zur Herstellung von N-Chlorcarbonyl-isocyaniddichlorid

Die vorliegende Erfindung betrifft ein neues Verfahren zur Herstellung von N-Chlorcarbonyl-isocyaniddichlorid durch Umsetzung von Methylisocyanat
mit Chlor.

N-Chlorcarbonyl-isocyaniddichlorid ist ein wertvolles Zwischenprodukt, das insbesondere zur Herstellung von Pflanzenschutzmitteln verwendet werden kann, z.B. indem man es mit Methanol zu Methoxycarbonyl-isocyaniddichlorid umsetzt und dieses mit
o-Phenylendiamin zum Benzimidazolcarbaminsäuremethylester. Dieser ist ein wertvolles Fungizid (siehe
DE-OS 1 900 755, DE-AS 1 932 297 und US-PS 2 593 504).

Bei dem besten bisher bekannt gewordenen Verfahren
zur Herstellung von N-Chlorcarbonyl-isocyaniddichlorid wird Methylisocyanat und/oder Methylcarbamidsäurechlorid in Lösung unter Einwirkung von ultra-

Le A 22 601

violettem Licht chloriert, wobei, je nach der Konzentration von Methylisocyanat im Reaktionsgemisch, bestimmte enge Temperaturbereiche einzuhalten sind (siehe DE-PS 2 503 168). Bei diesem Verfahren sind die erzielbaren Raum-Zeit-Ausbeuten nicht voll befriedigend und die Temperaturführung erfordert einen besonderen Aufwand.

Es wurde nun ein Verfahren zur Herstellung von N-Chlorcarbonyl-isocyaniddichlorid durch Umsetzung von Methylisocyanat mit Chlor in Gegenwart von Lösungsmitteln gefunden, daß dadurch gekennzeichnet ist, daß man die Umsetzung unter Zusatz von organischen, radikalbildenden Stoffen durchführt.

Methylisocyanat, das beim erfindungsgemäßen Verfahren als Ausgangprodukt verwendet wird, ist bekannt und kann z.B. aus Methylamin und Phosgen hergestellt werden, wobei man als Nebenprodukt gebildetes Methylcarbamidsäurechlorid durch Erhitzen auch in Methylisocyanat überführen kann.

Bei dem für das erfindungsgemäße Verfahren einzusetzenden Chlor kann es sich um Chlor in üblichen technischen Qualitäten handeln. Für die Umsetzung von einem Mol Methylisocyanat zu einem Mol N-Chlorcarbonyl-isocyaniddichlorid werden theoretisch 3 Mole Chlor benötigt. Man setzt deshalb zweckmäßigerweise mindestens 3 Mol Chlor pro 1 Mol Methylisocyanat ein. Vorzugsweise setzt man 3 bis 6 Mol, besonders bevorzugt 3 bis 5 Mol und ganz besonders bevorzugt 3,5 bis 4,5 Mol Chlor pro 1 Mol Methylisocyanat ein.

Das erfindungsgemäße Verfahren wird unter Zusatz von Lösungsmitteln durchgeführt. Geeignete Lösungsmittel sind beispielsweise chlorierte aliphatische Kohlenwasserstoffe, wie Methylenchlorid, Tetrachlorkohlenstoff und Trichlormethylisocyaniddichlorid. Vorzugsweise verwendet man als Lösungsmittel jedoch aus einem vorherigen Ansatz oder auf sonstige Weise erhaltenes N-Chlorcarbonyl-isocyaniddichlorid. Vorzugsweise sind bei Beginn der Reaktion von 93 bis 98 Gew.-% Lösungsmittel, bezogen auf das gesamte Reaktionsgemisch, zugegen.

Bei den erfindungsgemäß einzusetzenden organischen, radikalbildenden Stoffen kann es sich beispielsweise um organische Peroxide oder um Azobisisobutyronitril handeln. Bevorzugt sind Di-tertiär-butylperoxid, Dibenzoylperoxid, und seine chlorierten Derivate, wie 2,4-Dichlorbenzoylperoxid, und Dicumylperoxid. Die organischen, radikalbildenden Stoffe können zu Beginn der Reaktion beispielsweise in Mengen von 0,1 bis 5 Gew.-%, bezogen auf Methylisocyanat, vorliegen. Vorzugsweise beträgt diese Menge 0,5 bis 3 Gew.-%.

Das erfindungsgemäße Verfahren kann beispielsweise bei Temperaturen im Bereich von 80 bis 220°C durchgeführt werden. Bevorzugt sind Temperaturen im Be-

Le A 22 601

reich von 100 bis 180°C. Die Temperatur kann während der Reaktion konstant gehalten, aber auch innerhalb der angegebenen Bereiche variiert werden. Es ist weiterhin bevorzugt, die Reaktion bei solchen Temperaturen durchzuführen, bei denen die Halbwertzeit des jeweils eingesetzten organischen, radikalbildenden Stoffes im Bereich von 1 bis 30, vorzugsweise im Bereich von 1 bis 10 Minuten, liegt. Bei den bevorzugt einzusetzenden organischen, radikalbildenden Stoffen ergeben sich bei Berücksichtigung dieser Halbwertzeiten folgende Temperaturen: Beim Einsatz von Dibenzoylperoxid 100 bis 140°C, beim Einsatz von Di-tertiär-butylperoxid 140 bis 190°C und beim Einsatz von Dicumylperoxid 140 bis 180°C.

Die Zugabe der organischen, radikalbildenden Stoffe kann auf verschiedene Weise erfolgen. Wenn der einzusetzende organische, radikalbildende Stoff in reiner, trockener Form zur Verfügung steht kann er direkt oder im Gemisch mit einem Lösungsmittel, z.B. einem chlorierten aliphatischen Kohlenwasserstoff oder N-Chlorcarbonyl-isocyaniddichlorid oder im Gemisch mit Methylisocyanat in die Reaktion eingebracht werden. Wenn der einzusetzende organische, radikalbildende Stoff in wasserfeuchter Form zur Verfügung steht, muß er vor dem Einsatz in die Reaktion entwässert werden. Dies kann beispielsweise so durchgeführt werden, daß man den wasserfeuchten Stoff mit einem mit Wasser nicht mischbaren Lösungsmittel versetzt, z.B. einem chlorierten aliphatischen Kohlenwasserstoff, die sich bildende wässrige Phase abtrennt und aus der verbleibenden organischen Phase restliches Wasser durch Andestillieren entfernt. In diesen Fällen wird der organische, radikalbildende Stoff in Form des wasserfreien Gemisches mit dem Lösungsmittel in die Reaktion eingesetzt.

Das erfindungsgemäße Verfahren kann beispielsweise bei Drucken im Bereich von 1 bis 25 bar durchgeführt werden. Bevorzugt sind Drucke im Bereich von 1 bis 10 bar. Der jeweils gewünschte Druck wird vorzugsweise dadurch eingestellt, daß man die entweichenden Gase (im wesentlichen Chlorwasserstoff und gegebenenfalls überschüssiges Chlor) über ein entsprechend einreguliertes Drosselventil abnimmt.

Das erfindungsgemäße Verfahren kann kontinuierlich oder diskontinuierlich durchgeführt werden. Bei kontinuierlicher Arbeitsweise kann einstufig oder mehrstufig gearbeitet werden. Das Chlor kann zum flüssigen Reaktionsgemisch im Gleichstrom oder im Gegenstrom geführt werden. Das Chlor und/oder das Methylisocyanat können auch im Kreuzstrom zum flüssigen Reaktionsgemisch geführt werden. Bevorzugt ist die kontinuierliche, einstufige Arbeitsweise im Gleichstrom.

Zur Durchführung des erfindungsgemäßen Verfahrens eignen sich die verschiedensten Apparaturen, die zur Begasung von Flüssigkeiten üblich sind, beispielsweise Kessel, Rührkessel, Rührkesselkaskaden,

Le A 22 601

Blasensäulen oder Rieselfilmreaktoren. Diese Apparaturen sind mit Einrichtungen zur Gaseinleitung versehen, beispielsweise mit Einleitungsrohren, Düsen, Ejektoren, Brausen, Begasungsrührern oder Fritten. Die Apparate können gegebenenfalls Einbauten enthalten, beispielsweise Strömungsstörer oder Siebböden.

Die Aufarbeitung des nach der erfindungsgemäßen Umsetzung vorliegenden Reaktionsgemisches kann auf einfache Weise erfolgen. Zunächst wird während und/oder nach der Reaktion eine Entgasung durchgeführt, um Chlorwasserstoffgas und gegebenenfalls überschüssiges Chlor zu entfernen. Sofern N-Chlorcarbonyl-isocyaniddichlorid als Lösungsmittel verwendet wurde, liegt danach ein Produkt vor, das für weitere Umsetzungen schon genügend rein sein kann. Wenn andere Lösungsmittel als N-Chlorcarbonyl-isocyanidddichlorid verwendet wurden und/oder ein reineres Produkt gewünscht wird, kann man das Reaktionsgemisch destillieren. Das dabei abgetrennte Fremd-Lösungsmittel und/oder ein Teil des abgetrennten N-Chlorcarbonylisocyaniddichlorids kann in die Reaktion zurückgeführt werden.

Das erfindungsgemäße Verfahren liefert N-Chlorcarbonyl-isocyaniddichlorid in erheblichen höheren Raum-Zeit-Ausbeuten als das Verfahren gemäß der DE-PS 2 503 168.

Weiterhin ist das erfindungsgemäße Verfahren auf besonders einfache Weise durchführbar, da keine spezielle Temperaturführung nötig ist und es bereits bei einstufig-kontinuierlicher Fahrweise zu hervorragenden Ergebnissen führt.

Es ist ausgesprochen überraschend, daß diese Vorteile beim Einsatz von organischen, radikalbildenden Stoffen erhalten werden können, denn bei dieser Arbeitsweise, ist das gebildete N-Chlorcarbonyl-isocyaniddichlorid bei Reaktionstemperaturen wesentlich länger dem zersetzenden Einfluß von Radikalen ausgesetzt, als bei der Belichtung mit ultraviolettem Licht.

Die folgenden Beispiele erläutern das erfindungsgemäße Verfahren ohne es in irgendeiner Weise zu beschränken.

Beispiele .

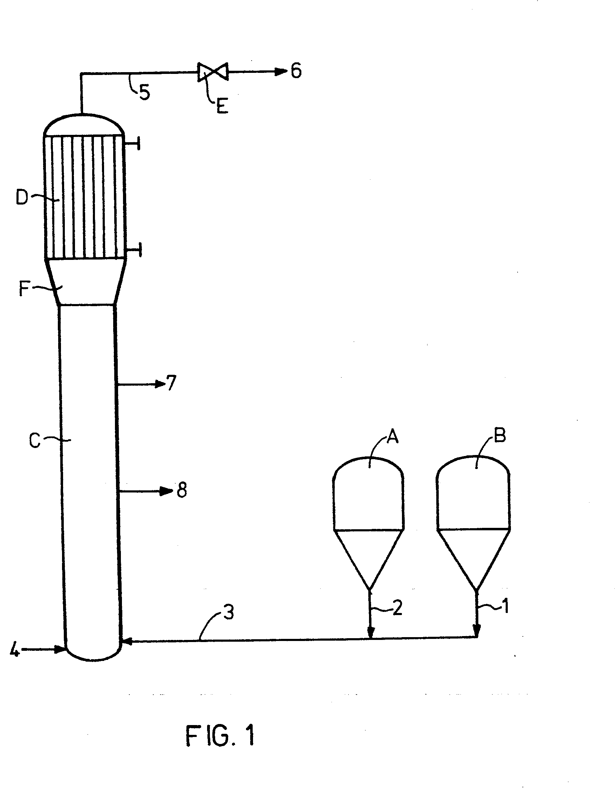

Beschreibung der verwendeten Apparatur (siehe Fig. 1)

Der Vorratsbehälter B enthält den organischen, radikalbildenden Stoff, gegebenenfalls im Gemisch mit Lösungsmittel. Der Vorratsbehälter A enthält Methylisocyanat, gegebenenfalls im Gemisch mit Lösungsmittel. Aus diesen Behältern, die auch zu einem Behälter zusammengefaßt sein können, werden Methylisocyanat, Lösungsmittel und der jeweilige organische, radikalbildende Stoff über die Leitungen 1, 2 und 3 und in der Figur nicht dargestellte Pumpen dem Reaktionsgefäß C zugeführt. Über die Leitung 4 wird dem Reaktionsgefäß C gasförmiges Chlor zugeführt. Das Reaktionsgefäß C hat ein Reaktionsvolumen von 10 l, ist zylindrisch gestaltet und am Boden mit einer Siebbodenplatte ausgestattet. Die Leitung 4 mündet unterhalb, die Leitung 3 oberhalb des Siebbodens in das Reaktionsgefäß. Über dem Reaktionsgefäß befindet sich ein Abstreifer F und ein Wärmeaustauscher D. Die den Wärmeaustauscher verlassende Gase werden über Leitung 5 dem Druckhalteventil E zugeführt und über Leitung 6 angenommen. Flüssiges Reaktionsprodukt wird über die Leitungen 7 oder 8 dem Reaktionsgefäß entnommen.

## Beispiel 1

Das Reaktionsgefäß C wurde mit 12 kg Tetrachlorkohlenstoff gefüllt und, unter Begasung mit 200 l/h Stickstoff durch Leitung 4, auf 120°C aufgeheizt, wobei das Druckhalteventil E so einreguliert wurde, daß sich im Reaktionsgefäß C ein Druck von 4 bar einstellte. Nach Erreichen der Solltemperatur wurde der Stickstoffstrom durch einen Chlorstrom von 1 kg/h ersetzt. Nachdem der Tetrachlorkohlenstoff mit Chlor gesättigt war, wurde über Leitung 3 pro Stunde ein Gemisch aus 854 g Methylisocyanat, 98 g Methylenchlorid und 25 g Dibenzoylperoxid in das Reaktionsgefäß eingepumpt und die Chlorzugabe auf 4,42 kg/h gesteigert. Der Füllstand im Reaktionsgefäß wurde durch Abnahme von flüssigem Reaktionsgemisch über Leitung 8 auf 10 l gehalten. Das abgenommene flüssige Reaktionsgemisch wurde abgekühlt und die im Verlaufe von 18 Stunden gesammelte Menge einschließlich Säuleninhalt durch eine 2 m-Raschigkolonne fraktioniert. Es wurden 41,8 kg 98,5 % reines N-Chlorcarbonyl-isocyaniddichlorid erhalten, was einer Ausbeute von 95,1 % der Theorie und einer Raum-Zeit-Ausbeute von 228 g/l.h entspricht. Die eingeleitete Menge Chlor betrug insgesamt 79,6 kg, das heißt 138 Gew.-% der theoretisch erforderlichen Menge.

## Beispiel 2

Es wurde verfahren wie in Beispiel 1, jedoch wurde das Reaktionsgefäß zu Beginn mit 12 kg N-Chlorcar-

Le A 22 601

bonyl-isocyaniddichlorid gefüllt, auf 170°C aufgeheizt und ein Druck von 6 bar eingestellt. Nach dem Umstellen der Stickstoffeinleitung auf Chlor wurden stündlich die 473 g Methylisocyanat und 15 g Di-tertiär-butylperoxid über Leitung 3 und 3,9 kg Chlor über Leitung 4 in das Reaktionsgefäß eingepumpt. Die destillative Aufarbeitung des im Verlauf von 17 Stunden gesammelten flüssigen Reaktionsgemisches ergab, nach Abzug der vorgelegten Menge, 19,43 kg N-Chlorcarbonyl-isocyaniddichlorid. Das entspricht einer Ausbeute von 85,8 % der Theorie und einer Raum-Zeit-Ausbeute von 114 g/l.h. Die eingeleitete Menge Chlor entsprach 220 Gew.-% der theoretisch erforderlichen Menge.

Beispiel 3

Es wurde verfahren wie in Beispiel 2, jedoch wurden nach dem Umstellen der Stickstoffeinleitung auf Chlor pro Stunde 485 g Methylisocyanat und 15 g Di-tertiär-butylperoxid über Leitung 3 und 2,62 kg Chlor über Leitung 4 eingeleitet. Die destillative Aufarbeitung des im Verlauf von 8 Stunden gesammelten flüssigen Reaktionsprodukts ergab, nach Abzug der vorgelegten Menge, 8,83 kg N-Chlorcarbonyl-isocyaniddichlorid. Das entspricht einer Ausbeute von 80,8 % der Theorie und einer Raum-Zeit-Ausbeute von 110 g/l.h. Die eingeleitete Menge Chlor entsprach 144 Gew.-% der theoretisch erforderlichen Menge.

Le A 22 601

Beispiel 4

Es wurde verfahren wie in Beispiel 2, jedoch wurde eine Temperatur von 160°C und ein Druck von 5 bar eingestellt. Nach dem Umstellen der Stickstoffeinleitung auf Chlor wurden stündlich 628 g Methylisocyanat und 30 g Dicumylperoxid über Leitung 3 und 3,85 kg Chlor über Leitung 4 eingeleitet. Die destillative Aufarbeitung des im Verlauf von 18 Stunden gesammelten flüssigen Reaktionsprodukts ergab, nach Abzug der vorgelegten Menge, 24,51 kg N-Chlorcarbonyl-isocyaniddichlorid. Das entspricht einer Ausbeute von 77 % der Theorie und einer Raum-Zeit-Ausbeute von 136 g/l.h. Die eingeleitete Menge Chlor entsprach 164 Gew.-% der theoretische erforderlichen Menge.

Patentansprüche

1. Verfahren zur Herstellung von N-Chlorcarbonyl-isocyaniddichlorid durch Umsetzung von Methylisocyanat mit Chlor unter Zusatz von Lösungsmitteln, dadurch gekennzeichnet, daß man die Umsetzung in Gegenwart eines organischen, radikalbildenden Stoffes durchführt.

2. Verfahren nach Anspruch 1, dadurch gekennzeichnet, daß man die Umsetzung in Gegenwart von organischen Peroxiden oder Azobisisobutyronitril durchführt.

3. Verfahren nach Ansprüchen 1 und 2, dadurch gekennzeichnet, daß zum Beginn der Umsetzung 0,1 bis 5 Gew.-% eines organischen, radikalbildenden Stoffes, bezogen auf Methylisocyanat, vorliegen.

4. Verfahren nach Ansprüchen 1 bis 3, dadurch gekennzeichnet, daß man die Umsetzung bei Temperaturen im Bereich von 80 bis 220°C durchführt.

5. Verfahren nach Ansprüchen 1 bis 4, dadurch gekennzeichnet, daß man die Umsetzung beim Einsatz von Dibenzoylperoxid bei 100 bis 140°C, beim Einsatz von Di-tertiär-butylperoxid bei 140 bis 190°C und beim Einsatz Dicumylperoxid bei 140 bis 180°C durchführt.

Le A 22 601

6. Verfahren nach Ansprüchen 1 bis 5, dadurch gekennzeichnet, daß man die Umsetzung bei Drucken von
1 bis 25 bar durchführt.

7. Verfahren nach Ansprüchen 1 bis 6, dadurch gekennzeichnet, daß man die Umsetzung in Gegenwart von
chlorierten aliphatischen Kohlenwasserstoffen
oder N-Chlorcarbonyl-isocyaniddichlorid durchführt.

8. Verfahren nach Ansprüchen 1 bis 7, dadurch gekennzeichnet, daß man bezogen auf 1 Mol Methylisocyanat 3 bis 6 Mole Chlor einsetzt.

9. Verfahren nach Ansprüchen 1 bis 8, dadurch gekennzeichnet, daß man die Umsetzung kontinuierlich,
in einstufiger Arbeitsweise und im Gleichstrom
von Chlor mit dem flüssigen Reaktionsgemisch
durchführt.

10. Verfahren nach Ansprüchen 1 bis 9, dadurch gekennzeichnet, daß man das Reaktionsgemisch aufarbeitet, indem man es zunächst entgast und anschließend destilliert.

FIG. 1